(19) Europäisches Patentamt
European Patent Office
Office européen des brevets

(11) Publication number: **0 051 355**
**A1**

(12) **EUROPEAN PATENT APPLICATION**

(21) Application number: 81304246.2

(22) Date of filing: 16.09.81

(51) Int. Cl.³: C 07 C 69/74
C 07 C 67/30, C 07 C 121/75
//A01N53/00

(30) Priority: 08.10.80 GB 8032459

(43) Date of publication of application:
12.05.82 Bulletin 82/19

(84) Designated Contracting States:
CH DE FR GB IT LI NL

(71) Applicant: IMPERIAL CHEMICAL INDUSTRIES PLC
Imperial Chemical House Millbank
London SW1P 3JF(GB)

(72) Inventor: Crosby, John
12 Oakwood Court Bowdon
Altrincham Cheshire WA14 3DJ(GB)

(74) Representative: Clark, Peter Frederick et al,
Imperial Chemical Industries PLC Legal Department,
Patents Thames House North Millbank
London SW1P 4QG(GB)

(54) **An improved process for the preparation of certain cyclopropane pyrethroid intermediates having a high cis-content.**

(57) "High *cis*" 3-[2,2-di(chloro or bromo)-3,3,3- trifluoropropyl]- or 3-[2,2,2-tri(chloro or bromo)ethyl]- 2,2-dimethylcyclopropane-1-carboxylates are obtained by reaction of the corresponding 3,3-dimethyl-4-halogenoheptanoate or -hexanoate with an alkali metal *tert*. alkoxide in a nitrile, a pyridine, an ester or a morpholine as solvent.

EP 0 051 355 A1

This invention relates to an improved process for the preparation of high cis 3-[2,2-di(chloro or bromo)-3,3,3-trifluoropropyl]- or 3-[2,2,2-tri(chloro or bromo)-ethyl]-2,2-dimethylcyclopropane-1-carboxylates which are valuable intermediates in the synthesis of pyrethroid insecticides.

Published European Patent Specification No.0003683 discloses a process whereby a compound of the formula:

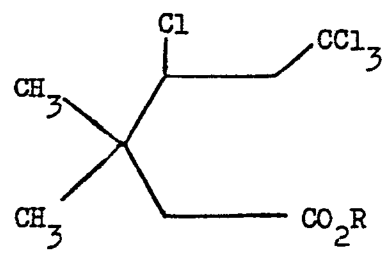

(B)

wherein R is defined as below, is treated with an alkali metal tert-alkoxide, preferably having 4 to 6 carbon atoms, at a temperature of about -80° to about 30°C in the presence of a solvent system comprising (a) a solvent selected from (1) an aliphatic hydrocarbon of 5 to 8 carbon atoms, (2) an aromatic unsubstituted hydrocarbon having 6 ring carbon atoms, or substituted with 1 to 3 substituents selected from alkyl of 1 or 2 carbon atoms and chlorine, (3) a tertiary alcohol of 4 to 6 carbon atoms, (4) an ether selected from diethyl ether, 1,2-dimethoxyethane, 2-methoxyethyl ether, tetrahydrofuran, and dioxane, and (5) a mixture of any of these solvents, and (b) a dipolar aprotic cosolvent selected from (1) hexamethylphosphoramide, (2) dimethylformamide, (3) dimethylacetamide, (4) dimethylsulfoxide, and (5) N-methylpyrrolidone, the ratio of solvent to cosolvent being from about 20:1 to about 2:1 by volume, to give a high cis compound of formula (Z)

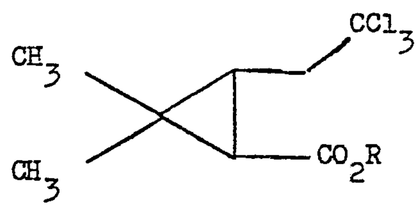

(Z)

wherein R is defined as below.

Treatment of high cis compound (Z) with base produces a high cis compound of formula (C).

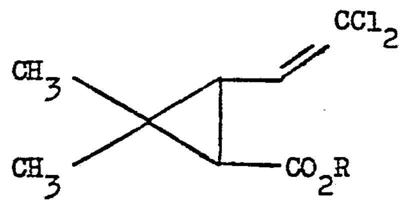

(C)

wherein R is defined as below.

In the above formulae, R is inter alia lower alkyl, 3-phenoxybenzyl, α-cyano-3-phenoxybenzyl or 5-benzyl-3-furylmethyl.

Throughout this specification the term "lower alkyl" means an alkyl group containing from 1 to 4 carbon atoms.

The high cis compounds of formula (C) above wherein R is, for example, 3-phenoxybenzyl, for which the compounds of formula (Z) are intermediates, are highly active insecticides. It is well known that cis pyrethroids are generally more toxic to insects than are the corresponding trans compounds. (M.Elliott, Ed., Synthetic Pyrethroids, ACS Symposium Series, No.42, American Chemical Society, Washington D.C., 1977, p.53).

The examples illustrating the invention disclosed in published European Patent Specification No.0003683 show the preparation of compounds of formula (Z) in which the

cis/trans ratio varies from 53/47 to 81/19. Whilst this prior art process is therefore effective in providing compounds of formula (Z) predominantly in their cis isomer form, the use such a mixed solvent system gives rise to difficulties in solvent recovery and recycle, and effluent disposal, in large scale operation. It is therefore desirable to prepare compounds of formula (Z) from compounds of formula (B) by means of a process which reduces and preferably eliminates these difficulties without sacrificing the high cis characteristics of the known method. The present invention provides such a process.

According to the present invention there is provided a process for the preparation of a compound of formula (I):

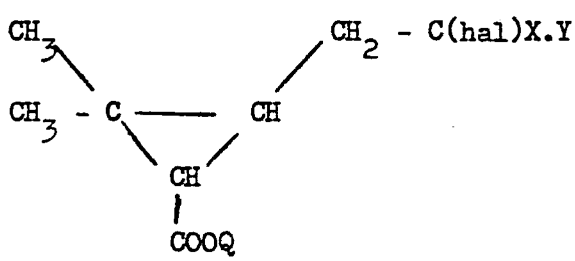

(I)

wherein Q is lower alkyl, 3-phenoxybenzyl, α-cyano-3-phenoxybenzyl or α-ethynyl-3-phenoxybenzyl, hal is chlorine or bromine, and each of X and Y is chlorine or bromine, or one of X and Y is chlorine or bromine and the other is a group of the formula $-(CF_2)_mW$ in which W is hydrogen, chlorine or fluorine and m is 1 or 2, which comprises treating a compound of formula (II):

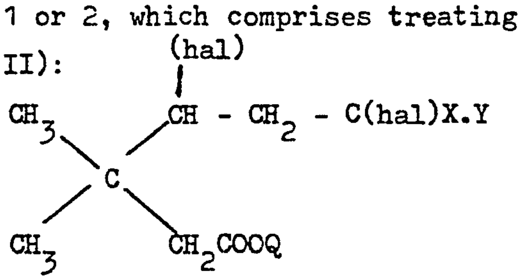

(II)

wherein hal, Q, X and Y have the meanings stated above, with an alkali metal tert-alkoxide preferably containing from 4 to 6 carbon atoms at a temperature below about 30°C and in the presence of a solvent which is substantially inert towards the reactants and which gives a stirrable reaction mixture at the reaction temperature, the solvent being selected from nitriles, pyridines, esters and morpholines.

Examples of the alkali metal tert-alkoxides which may be used are sodium or potassium tert.butoxide or tert.pentyloxide. The amount of alkali metal tert-alkoxide which is used may be from 1.0 to 3.0 mol.per mol. of the compound of formula (II).

In addition to the requirements specified above, the solvent should also preferably be such that the alkali metal tert-alkoxide is at least partially in solution, although phase transfer techniques may be employed if desired. Total dissolution of the alkoxide is not essential. The solvent should preferably have a boiling point at atmospheric pressure not exceeding 150°C, since such solvents are easily removable from the reaction system, for example, by distillation under normal or moderately reduced pressure.

The nitrile solvents which may be used in the process may be of the aliphatic or aromatic series, and examples of suitable nitriles are acetonitrile, propionitrile, isobutyronitrile, benzonitrile and o-, m- and p-tolunitrile. Secondary and tertiary nitriles are preferred because primary nitriles may give rise to undesirable by-products in the presence of the strongly basic alkali metal tert.alkoxide.

Examples of the pyridines which may be used are pyridine itself, substituted pyridines such as the isomeric methylpyridines (picolines) and fused pyridines such as quinoline.

The esters which may be used as solvents are preferably such that the alcohols from which they are derived are not of lower pKa value than the alcohol from which the alkali metal tert-alkoxide is derived, so that any ester interchange which may occur between the ester and the alkali metal tert-alkoxide will not result in in situ generation of an alkoxide which is a weaker base than the latter. For this reason esters of tertiary aliphatic alcohols are preferred, examples of such esters being tert.butyl acetate, tert.pentyl acetate, tert.butyl propionate and tert.pentyl propionate.

The morpholines which may be used as solvents include morpholine itself and, for example, simple substituted derivatives such as 2- or 3-alkyl or aryl morpholines.

The amount of solvent which is used is not critical, but must be sufficient to give an easily stirrable reaction mixture.

It is preferred that the reaction temperature does not exceed 25°C, and further preferred that it does not exceed 0°C. Reaction is conveniently carried out at -10°C, a temperature which is readily attainable under large scale manufacturing conditions without resort to special refrigeration units.

In general, the process of the invention may be carried out by adding the compound of formula (II) to a mixture of the solvent and the alkali metal tert-alkoxide, with stirring and under an atmosphere of dry nitrogen, whilst maintaining the desired reaction temperature. The progress of the reaction is easily monitored by GLC analysis of the reaction mixture. When all of the compound of

formula (II) has been consumed, the mixture may conveniently be distilled to leave a mixture of crude product plus salt. The reaction product of formula (I) may be isolated by solvent extraction or by simple precipitation with water. It may then be purified by conventional means, for example, distillation, steam distillation or recrystallisation. Alternatively, the product may be used in the crude form for the preparation of the pyrethroid acid of formula (III):

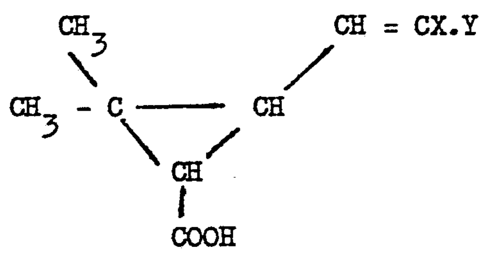

(III)

wherein X and Y have the previously defined meanings, by, for example, heating the crude compound of formula (I) with aqueous alcoholic sodium hydroxide, removal of the alcohol, e.g. by distillation, and acidification to give crude (III). The latter typically has a cis/trans ratio of 85-90:15-10. Recrystallisation of the crude acid readily enhances the cis content. For example, recrystallisation from toluene will generally give a product containing 95-100% of the desired cis isomer.

The initial part of the process may also be carried out by adding a solution or slurry of the alkali metal tert-alkoxide in the solvent to the compound of formula (II), or by adding the neat alkali metal tert-alkoxide to a solution of the compound of formula (II) in the solvent, the reaction then being carried out as before.

The invention is illustrated by the following Examples in which percentages are by weight.

Example 1

Potassium tert-butoxide (15.25 g) was added to pyridine (175 ml) with stirring, under an atmosphere of dry nitrogen, and cooled to -10°C. Methyl 3,3-dimethyl-4,6,6-trichloro-7,7,7-trifluoroheptanoate (36.6 g) was then added slowly, with vigorous stirring, whilst maintaining the temperature of the reaction mass at -10° to -12°C. The reaction was monitored by following the disappearance of the heptanoate by glc (5', 5% ov 17 column operated at 140°C). When all the heptanoate had been added, further aliquots of potassium tert-butoxide were added until it was all consumed. The reaction mixture was then drowned into a large volume of water, extracted with toluene, and the organic extract washed with dilute hydrochloric acid and evaporated under reduced pressure.

The resulting crude ethyl 3-(2',2'-dichloro-3',3',3'-trifluoroprop-1'-yl)-2,2-dimethylcyclopropanecarboxylate was added to a solution of sodium hydroxide (11.2 g) in a mixture of water (16 ml) and ethanol (160 ml) and heated at reflux for 67 hours. At the end of the reflux period the bulk of the ethanol was distilled off the reaction mixture. The mixture was diluted with water, extracted with dichloromethane to remove neutral impurities, and acidified with concentrated hydrochloric acid. The resulting 3-(2'-chloro-3',3',3'-trifluoroprop-1'-enyl)-2,2-dimethylcyclopropanecarboxylate was extracted into dichloromethane, dried (anhydrous sodium sulphate) and evaporated to afford 24.2 g of product containing the cis Z, cis E, trans Z, and trans E * isomers in the ratios 80:3:10:7 (by 'H nmr analysis); glc analysis showed the strength of the cis Z isomer to be 56% wt/wt of the product.

*cis/trans refers to the geometry of the cyclopropane ring, Z/E refers to the geometry of the propenyl side chain.

Example 2

Methyl 3,3-dimethyl-4,6,6-trichloro-7,7,7-trifluoroheptanoate (36.6 g) in pyridine (100 ml) was stirred under an atmosphere of dry nitrogen and cooled to -10°C. Potassium tert-butoxide (15.25 g) in further pyridine (200 ml) was then added slowly with vigorous stirring whilst maintaining the temperature at -10° to -12°C; the reaction was monitored as in Example 1 and further aliquots of butoxide added until all the heptanoate had reacted. The reaction mixture was then worked up and treated with aqueous ethanolic sodium hydroxide solution as in Example 1 to afford 24.4 g of product containing the cis Z, cis E, trans Z and trans E isomers in the ratios 72:6:15:7 ('Hnmr analysis). Recrystallisation from hexane afforded 12.2 g of material containing 95% (wt/wt) of the cis Z isomer.

Example 3

The method of Example 2 was repeated except that the solvent was acetonitrile and the hydrochloric acid wash was omitted from the work-up procedure. Using a hydrolysis time of 41.5 hours, crude 3-(2'-chloro-3',3',3'-trifluoroprop-1'-enyl)-2,2-dimethylcyclopropanecarboxylate (21.8 g) was obtained having the cis Z, cis E and trans (E + Z) isomers in the ratios 59:13:28 and containing 46.6% wt/wt of the cis Z isomer.

Example 4

The method of Example 1 was repeated except that the solvent was acetonitrile and the hydrochloric acid wash was omitted from the work-up procedure. Using a hydrolysis time of 69 hours crude carboxylic acid (21.0 g) was obtained

having the cis Z, cis E, trans Z and trans E isomers in the ratios 78:3.5:11.5:7 and containing 61.8% wt/wt of the cis Z isomer.

Further examples of the invention are given in the following table.

| Ex. | (a) Substrate | (b) Scale (g.) | Solvent | Method as in Example | Period of reflux with aqueous ethanolic NaOH (hours) | Isomer Ratios (c) | | | |
|---|---|---|---|---|---|---|---|---|---|
| | | | | | | Cis Z | Cis E | Trans Z | Trans E |
| 5 | M | 6.6 | Pyridine | 1 | 48 | 82.5 | 4 | 7.5 | 6 |
| 6 | M | 6.6 | Acetonitrile | 4 | 48 | 82 | 6 | (E+Z) 12 | |
| 7 | M | 3.3 | Morpholine | 1 | 48 | 71 | 8 | (E+Z) 21 | |
| 8 | M | 6.6 | 4-Picoline | 1 | 48 | 84 | 9 | (E+Z) 7 | |
| 9 | E | 6.6 | iso-butyronitrile | 4 | 48 | 84 | 8 | (E+Z) 8 | |
| 10 | E | 6.6 | Benzonitrile | 4 | 40 | 82 | 9 | (E+Z) 10 | |
| 11 | E | 6.6 | Propionitrile | 4 | 67 | 88 | 6 | (E+Z) 6 | |
| 12 | E | 6.6 | tert-butylacetate | 4 | 45 | 80 | 10 | (E+Z) 10 | |
| 13 | E | 6.6 | Quinoline | 1 | 45.5 | 75 | 11 | 4 | 10 |
| 14 | E | 38.2 | iso-butyronitrile | 4 | 48 | (E+Z) 89 | | (E+Z) 11 | |
| 15 | E | 38.2 | Propionitrile | 4 | 40.5 | 86 | 6.5 | 2.5 | 5 |

(a): M = methyl 3,3-dimethyl-4,6,6-trichloro-7,7,7-trifluoroheptanoate;

E = ethyl 3,3-dimethyl-4,6,6-trichloro-7,7,7-trifluoroheptanoate.

(b): amount of substrate used in the reaction.

(c): 'Hnmr analysis of the proportions of the four isomers of 3-(2'-chloro-3',3',3'-trifluoroprop-1'-enyl)-2,2-dimethylcyclopropanecarboxylate in the crude product (i.e. before any recrystallisation) obtained after treating the crude ethyl (or methyl) 3-(2',2'-dichloro-3',3',3'-trifluoroprop-1'-yl)-2,2-dimethylcyclopropanecarboxylate first with aqueous ethanolic sodium hydroxide and then with acid.

CLAIMS

1. A process for the preparation of a compound of formula (I):

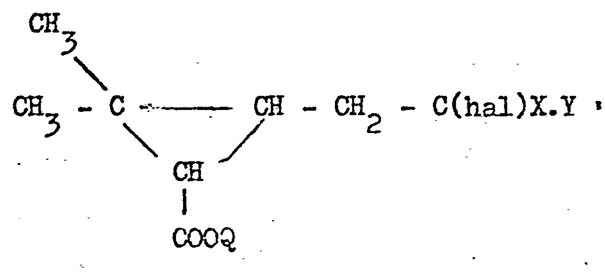

(I)

wherein Q is lower alkyl, 3-phenoxybenzyl, α-cyano-3-phenoxybenzyl or α-ethynyl-3-phenoxybenzyl, hal is chlorine or bromine, and each of X and Y is chlorine or bromine, or one of X and Y is chlorine or bromine and the other is a group of the formula $-(CF_2)_mW$ in which W is hydrogen, chlorine or fluorine and *m* is 1 or 2, which comprises treating a compound of formula (II):

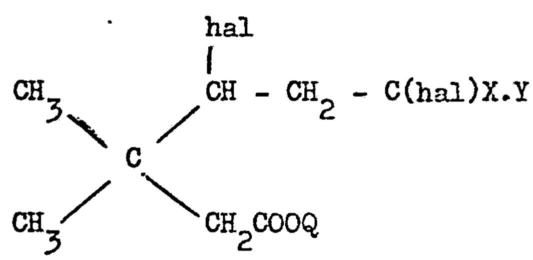

(II)

wherein hal, Q, X and Y have the meanings stated above, with an alkali metal tert.alkoxide at a temperature below about 30°C and in the presence of a solvent which is substantially inert towards the reactants and which gives a stirrable reaction mixture at the reaction temperature, the solvent being selected from nitriles, pyridines, esters and morpholines.

2. A process as claimed in claim 1 wherein the alkali metal tert.alkoxide contains from 4 to 6 carbon atoms.

3. A process as claimed in claim 1 or claim 2 wherein the amount of alkali metal tert.alkoxide used is from 1.0 to 3.0 mols per mol of the compound of formula (II).

4. A process as claimed in any one of claims 1 to 3 wherein the solvent is such that the alkali metal tert. alkoxide is at least partially in solution.

5. A process as claimed in any one of claims 1 to 4 wherein the solvent has a boiling point at atmospheric pressure not exceeding 150°C.

6. A process as claimed in any one of claims 1 to 5 wherein the nitrile solvent is a secondary or tertiary nitrile.

7. A process as claimed in any one of claims 1 to 5 wherein the ester solvent is the ester of a tertiary aliphatic alcohol.

8. A process as claimed in any one of claims 1 to 7 wherein the reaction temperature does not exceed 0°C.

9. A process as claimed in claim 8 wherein the reaction temperature is -10°C.

PFC/BH
14.9.81.

0051355

European Patent Office

# EUROPEAN SEARCH REPORT

Application number
EP 81 30 4246.2

## DOCUMENTS CONSIDERED TO BE RELEVANT

| Category | Citation of document with indication, where appropriate, of relevant passages | Relevant to claim |
|---|---|---|
| D | EP - A2 - 0 003 683 (FMC)<br>* claims 1,6 *<br>-- | 1-3 |
| | FR - A1 - 2 322 845 (ICI)<br>* page 6, lines 4 to 14; example 3 *<br>-- | 1 |
| | FR - A1 - 2 333 776 (ICI)<br>* claim 1; page 6, lines 4 to 14 *<br>---- | 1 |

CLASSIFICATION OF THE APPLICATION (Int. Cl.[3])

C 07 C 69/74
C 07 C 67/30
C 07 C 121/75
//A 01 N 53/00

TECHNICAL FIELDS SEARCHED (Int. Cl.[3])

C 07 C 67/30
C 07 C 69/74
C 07 C 121/75

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 11-01-1982 | KNAACK |

EPO Form 1503.1 06.78